# EUROPEAN PATENT APPLICATION

(11) **EP 1 082 940 A1**
(43) Date of publication of application: **14.03.2001**
(21) Application number: 99921181.6
(22) Date of filing: 19.05.1999
(51) Int. Cl.: A61B 5/00

(54) **IONTOPHORESIS DEVICE STRUCTURAL BODY AND METHOD FOR DETECTING IN-VIVO COMPONENT**

(30) Priority: 05.06.1998 JP 17402498
(71) Applicant: HISAMITSU PHARMACEUTICAL CO. INC., Tosu-shi Saga 841-0017 (JP)
(72) Inventor: HIGO, Naruhito Tsukuba Lab.Hisamitsu Pharm.Co.Inc, Tsukuba-shi Ibaraki 305-0856 (JP); ADACHI, Hirotoshi Tsukuba Lab.Hisami.Pharm.Co.Inc, Tsukuba-shi Ibaraki 305-0856 (JP); KUZUMAKI, Noriyuki Tsukub.Lab.Hisami.Pharm.Co.Inc, Tsukuba-shi Ibaraki 305-0856 (JP)
(74) Representative: Splanemann Reitzner Baronetzky Westendorp Patentanwälte
(86) International application number: JP9902623
(87) International publication number: WO9963885

(57) **Abstract**

An iontophoresis device structure and a detecting method in which a physiological substance can be detected rapidly and in high sensitivity, are provided. In this structure, an electrode (2) is set in the bottom of a backing (1) having a concavity, and a conductive layer (3) is arranged in the concavity part. In the electrode (2), a connection terminal (4) is fixed through a small hole opened in the bottom of the backing (1) so as to be connectable with a power source. In such structure a member (6) for detection constituted so as to adsorb a physiological substance is arranged. The member (6) for detection is attached to a sticky sheet (5) where an opening is preset in the corresponding part. The member (6) for detection is used so as to be in contact with the skin or mucous membrane when starting the iontophoresis.

## Description

### TECHNICAL FIELD

The present invention relates to an iontophoresis device structure and a method for detecting a physiological substance suitable to be used in diagnosis and examination on the medical field.

### BACKGROUND ART

Various methods have been used for diagnosis of diseases. For example, biochemical examination of blood where diagnose is performed by analyzing blood components, a variety of diagnostic markers, cells and the like after invasively collecting blood, pathological examination where vital histological examination of cancer tissues etc. is invasively performed or examined whether cell levels are normal or not, and examinations by analyzing urine, stool, saliva, etc. after collection of urine, stool, saliva, etc. In these methods in the present state, medical doctors, nurses or clinical technologists collect blood etc. by injectors or cut off living tissues by a sharp scalpel or collect urine etc. in a specific container in cooperation with patients.

Since a tumor, particularly a malignant melanoma is a disease which has an extremely poor prognosis and has both extremely high madidans destruction ability and metastability. Once the metastasis or recurrence occurs, its treatment is highly difficult and therefore, development of new therapeutic means is expected. The definite diagnosis of such malignant melanoma is performed by examining whether or not tumor markers such as melanoma cells in surgically excised tissue exist. As the examination methods, the stamp fluorescence method which uses stamp smears prepared from the lesion section immediately after surgery, and the immuno staining technique which uses lesion tissues, are used. For the tissue staining, a variety of melanoma marker antibodies (antimelanoma antibodies) are used.

Such a surgical excision of cells, however, is not desirable because it increases risk of metastasis of the tumor besides it gives patients pain and scare. Further, since collection of samples must be performed by skilled medical doctors at a specified place, it is not suitable for a screening method in mass health examination and the like. In addition, although measurement of DOPA such as 5-S-cystenyldopa and tumor markers in serum and urine provides indexes of disease condition such as progressive degree at deteriorated stages of the symptoms and whether the metastasis exists or not, it is not appropriate for the early diagnostic method. Under such a situation, development of the method is expected in which high level skill and experience necessary in the conventional methods are not required so much and early diagnosis of tumors etc. can be safely performed.

On the other hand, the following conventional plasters are known: medical plasters containing medicines such as antiphlogistic analgesics of salicylic acids and therapeutic agents of nitroglycerin etc. for angina pectoris; and plasters for patch tests of irritation and for detecting allergens in order to identify sensitizers in allergic dermatitis and the like. In Japanese Patent Laid-Open No. 63-106558, preparation of stratum corneum samples comprising the following methods is disclosed: after sticking dermal stratum corneum onto a sticky material, this is attached on a transparent plate on which a sticking agent is applied, then the above described sticky material is dissolved and removed by immersing this plate in organic solvents to fix the above described stratum corneum on the above described transparent plate. Further in Japanese Patent Laid-Open No. 63-113358 by the same applicant, a method for examining the state of the stratum corneum using the above described stratum corneum samples is disclosed in which the examinations performed are mainly the ones by measuring physical property of the stratum. Furthermore, in Japanese Patent Laid-Open No. 4-121664 by the same applicant, the following methods are disclosed: stratum corneum is detached from a sticky film and transferred; then it is washed with organic solvents; and a lipid contained in the stratum corneum is extracted followed by analyzing it.

However, in the methods by the above described Japanese Patent Laid-Open No. 63-106558 and Japanese Patent Laid-Open No. 63-113358, it is necessary to dissolve the adhesive layer in organic solvents and the like to fix the detached corneocytes. Therefore, unstable materials to organic solvents can not be examined. In addition, no materials to be extracted from organic solvents like a stratum corneum lipid exist in the stratum corneum sample, and therefore, it is impossible to examine the stratum corneum in the same state as that in the skin. In the methods of the above described Japanese Patent Laid-Open No. 4-121664, it is also impossible to analyze materials which are not extracted from organic solvents.

In Japanese Patent Laid-Open No. 7-76518, a diagnosing method of diseases is disclosed in which it is performed by measuring the tissues and cells or other materials detached with a sticky tape etc. and fixed, or materials secreted from tissues or cells adsorbed and fixed on a carrier and the like by the immunological method etc. In early stage of a tumor, particularly malignant melanoma, this method does not need surgical excision of tissues etc., and diagnosis of the tumors can be performed collecting vital tissues, cells, or secretion products from them, or the like without giving patients pain and scare, and the like;

An iontophoresis is an accelerating system for transdermal absorption using electricity as an outside stimulus. The principal is that it accelerates penetration of the drug's molecules through the skin barrier based on the moving power of positively charged molecules from the anode to the cathode and negatively charged molecules from the cathode to the anode, in the electric field generated between the anode and the cathode mainly by energizing with current (see Journal of Controlled Release; vol. 18, 213-220, 1992; Advanced Drug Delivery Review, vol. 9, 119, 1992; Pharmaceutical Research, vol. 3, 318-326, 1986).

As an applied example to diagnosis using such an iontophoresis, administration of pilocarpine is known for the purpose of diagnosis of cystic fibrosis (see J. Pediatr, vol. 69, 285, 1966; South Med. J., vol. 59, 197, 1966; Arch. Dis. Child, vol. 40, 684. 1965). Recently, self measurement of blood sugar of diabetics, a monitoring technique of glucose in blood using a noninvasive iontophoresis is proposed (WO No. 96/00110; Pharmaceutical Research, vol. 12, 1869-1873, 1995; Journal of Controlled Release, vol. 38, 159-165, 1996; Ibid., vol. 42, 29-36, 1996). This method is that glucose leaked from blood is transferred to a solution layer set up in a device by convective flow generated by an iontophoresis to perform sampling.

In the method using an adhesive tape etc. among the conventional methods, adhesion for long time is required particularly when collecting secretion from living tissues and cells, which are polymer materials such as peptides and proteins, therefore the compliance may not be satisfied. Further, to the skin stimulation due to adhesion for long time is also concerned, resulting in giving patients uneasy and uncomfortable feeling. In addition, depending on only passive diffusion like this method increases scattering among individuals causing incorrect diagnosis, therefore it may not be adequate in terms of performance.

Furthermore, in the methods using the conventional iontophoreses, detection of lower molecular materials is possible, however, they are poor ly practical in terms of measuring sensitivity when detecting polymer materials such as peptides and proteins, because not only the complex operations are required to recover the measured materials to be measured, but also they are diluted in the sampling solution.

Thus, there have not been devices and methods of detection where the desired substances etc. can be detected rapidly and in high sensitivity so far. This leaves problems that correct diagnoses in short time can not be performed on some kinds of diseases.

Accordingly, an object of the present invention is to provide an iontophoresis device structure and a detecting method in which a physiological substance can be detected rapidly and in high sensitivity.

### DISCLOSURE OF THE INVENTION

The present inventors have assiduously studied on an iontophoresis device structure to achieve the above purposes. As a result, it was found that the above problems could be dissolved by use of a member for detection as a device structure that was constituted so as to suitably adsorb a physiological substance.

Specifically in the case of diagnosis of diseases such as tumors, for example, an iontophoresis is performed using a device structure for diagnosis which arranges a member for detection showing high adsorption to a protein, a peptide, a nucleotide, etc. which are markers of tumors and the like. Further, the physiological substance is adsorbed and fixed onto the member for detection by electrically driving power, then the member for detection is detached from the device, and the tumor-related antigens, tumor markers or tumor-related substances adsorbed and fixed are measured by immunological or chemical method or the like.

Briefly, the iontophoresis device structure according to the present invention comprises electrodes for an iontophoresis, a member for detection which is constituted so as to adsorb a physiological substance by an iontophoresis; and a conductive layer arranged between the above described electrodes and the member for detection. Herein, the member for detection, for example, is composed of a porous membrane where the adsorbability to protein shows high adsorption of 20µg per cm² or more. In addition, thickness of the member for detection is desirably 5-200µm.

The member for detection is constituted so as to adsorb at least one of living tissues, blood and cells as physiological substances or so as to adsorb a substance secreted from at least one of vital tissues, blood and cells. Such secretion is, for example, a peptide or a protein.

Further, the member for detection is constituted so as to adsorb tumor-related antigens, tumor markers or other tumor-related substances. Herein, the tumor-related antigens, tumor markers or other tumor-related substances are selected from groups composed of melanoma cells, malanoma marker (NKI/C3), melanoma marker (PAL/M1), melanoma markers (S-100 α and β), carcinoembryonic antigen (CEA), neuroblastoma (CE 7), neuroblastoma (AD2), maiignin, α-fetoprotein (AFP), pepsinogen, basic fetoprotein (BFP), pancreas carcinoembryonic antigen (POA), embryonic prealbumin (EPA), carbohydrate antigen (CA19-9), pancreatic cancer-related antigen (CA50), cancer antigen (CSLEX-1), pancreatic cancer-related antigen (sialylSSEA- 1), pancreatic cancer-related antigen (Dupan-2), cancer antigen (NCC-ST-439), carbohydrate antigen (sialylTn), cancer antigen (CA72-4), cancer antigen (KMO-1), pancreatic cancer-related antigen (SPan-1), carbohydrate antigen (CA125), cancer antigen (CA15-3), planocellular carcinoma (SCC), seminoprotein (γ-Sm), prostatic specific antigen (PA), ferritin, tissue polypeptide antigen (TPA), tumor-related antigen (CYFRA-21-1), acidic immunoprotein (IAP), immunosuppressive acidic protein, prostatic acidic protein (PAP), neuron specific enolase (NSE), chorionicgonadotropin (hCG), enzyme, amino acid, mucosa containing mucopolysaccharide. dopa, dopamine and hormones.

An applicator for an iontophoresis according to the present invention is configured by being provided with a backing having a concavity, electrodes arranged in the bottom of a backing concavity, a member for detection of a physiological substance arranged in the top of a backing concave, and a conductive layer arranged between the electrodes and the member for detection of a physiological substance. An adhesive sheet having an opening is provided in the member corresponding to the member for detection of a physiological substance.

In the detecting method of a physiological substance according to the present invention, a physiological substance is adsorbed onto the member for detection using the iontophoresis and the physiological substance adsorbed onto the member for this detection are detected by an immunological or chemical method. Such detection is performed by staining or measuring of the physiological substance.

The iontophoresis system according to the present invention is configured by being provided with a member for detection which is constituted so as to adsorb a physiological substance by iontophoresis, electrodes for a device, an iontophoresis device containing a conductive layer arranged between the electrodes for the device and the member for detection, a control device containing reference electrodes set corresponding to the electrodes for the device, and a power source connecting electrically between the above described electrodes for the device and reference electrodes. Herein, the member for detection, for example, has a porous structure of the average pore size of 0.001-20µm.

From the above system, the iontophoresis device of the present invention can detect the physiological substance rapidly and in high sensitivity, being extremely useful for diagnoses of various diseases.

### BRIEF DESCRIPTION OF THE DRAWINGS

FIG. 1 (a) is a cross section showing an example of an applicator provided with an iontophoresis device structure according to the present invention, and (b) is a conceptional view showing an example of an iontopboresis system according to the present invention.
FIG. 2 is a graph showing adsorbability of bovine serum albumin to a member for detection to be used as a device for diagnosis.
FIG. 3 is a graph showing adsorbability of human insulin to a member for detection to be used as a device for diagnosis.
FIG. 4 (a)-(d) each are views showing the results of immunological staining of human carcinoembryonic antigens on the member for detection.

### BEST MODE FOR CARRYING OUT THE INVENTION

Hereinafter, the present invention will be described in detail referring to the drawings.

The iontophoresis device structure according to the present invention comprises electrodes for an iontophoresis, a member for detection which is constituted so as to adsorb a physiological substance or ingredient by an iontophoresis, and a conductive layer arranged between the above described electrodes and the member for detection. Further, using various applicators applicable to the skin or mucous membrane, physiological substances moved by the iontophoresis are detected by an immunological or chemical method.

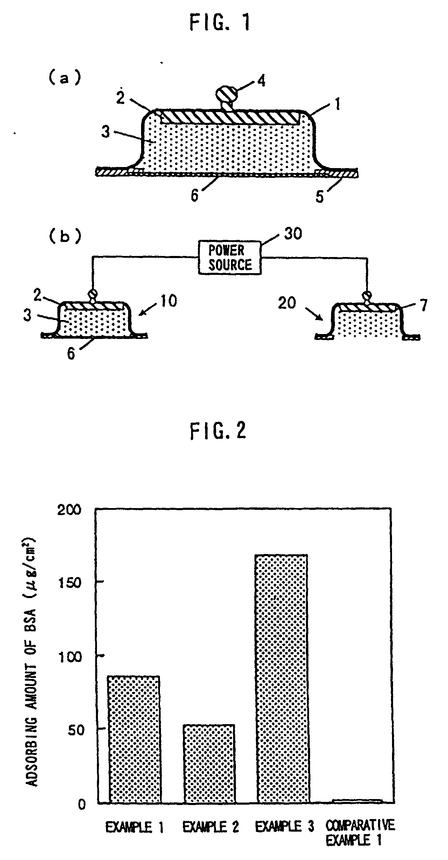

FIG. 1 (a) is a cross section showing an example of an applicator provided with an iontophoresis device structure according to the present invention. As shown in the figure backing 1 forms a cylindrical concave. As the backing 1, for example, the polypropylene one having an inside diameter of 18 mm is used. A foil type silver electrode 2 where a hole is circularly punched is set in the bottom of backing 1. The foil type silver electrode 2 has, for example, a diameter of 10 mm and a thickness of 0.04 mm. In the concave of backing 1, an electrolyte layer is arranged as a conductive layer 3. Also in the foil type silver electrode 2, a connection terminal 4 is fixed through a small hole opened in the center of the backing 1 so as to be connectable with a power source. On the device constituted in such way, a member 6 for detection constituted so as to adsorb a physiological substance is arranged. Although the materials of the member 6 for detection will be described in detail later, for example, a hydrophilic nylon membrane having a diameter of 20 mm and a thickness of 0.016 mm is used. The member 6 for detection is in advance attached to an adhesive sheet 5 having an opening of 18 mm in diameter slightly smaller than that of the sheet, and the member 6 for detection is used so as to be in contact with the skin and mucous membrane when starting the iontophoresis.

A shape of the iontophoresis device structure according to the present invention may be the any ones, for example, including a rectangle, a circle, an elliptical, a square, a heart shape, a lozenge or the like, but not limited to them. Neither particularly limited the size and color, if accompanied with practical use.

Energizing with current of the iontophoresis in the present invention, as shown in FIG. 1 (b), can be performed by applying direct voltage using a power source 30 between electrode 2 of device 10 and reference electrode 7 of control device 20 set corresponding to it. The power source which can apply the continuous direct voltage or the pulse direct voltage is preferably used, and the one which apply the rectangle pulse directive voltage is more preferably used. The frequency of the pulse direct voltage is optionally selected from the range of preferably 0.1 to 200 kHz, more preferably 1 to 100 kHz, most preferably 5 to 80 kHz. On/off ratio of the pulse direct voltage is optionally selected from the range of 1/100 to 20/1, preferably, 1/50 to 15/1, more preferably, 1/30 to 10/1. Energizing time with current is not more than 24 hr in continuous energizing with current, preferably not more than 12 hr, most preferably not more than 6 hr.

In such an iontophoresis device, for example, if the substance to be detected is a protein close to 4 of an isoelectric point, it will have a negative charge in the living body such as the skin and mucous membrane, resulting in moving to the anodic side in application of the iontophoresis. Thus, the device for detection will be arranged to the anodic side. After energizing the iontophoresis device with current, the living tissues, blood and cells or substances secreted from those which are adsorbed and fixed onto the member for detection, are detected by an immunological or chemical method, or the like.

In the device structure according to the present invention, although a reinforcing method of the structure, that is, backing is not particularly limited, if it is an electrical insulator or an insulation-treated one, the following methods are preferably used: using a plastic film such as vinyl chloride, polyethylene, polyolefin, polyester, polyurethane, polyvinyl alcohol, vinylidene chloride polyamide and ethylene-vinyl acetate copolymer; or a laminate film containing aluminum or the like. Although thickness of the backing is not particularly limited, it is preferably about 1µm-5000µm, particularly about 10µm-600µm.

The electrodes used in the present invention are not particularly limited if they are conductive materials for electrode which can be usually used for iontophoresis. Such conductive materials include, e.g. silver, silver chloride, aluminum, zinc, copper, iron, carbon, platinum, titanium, stainless, or the like. Among them, silver or silver/silver chloride has good electrical properties such as a resistance value as well as high manufacturing efficiency with a low cost when manufacturing with a paste material.

The conductive layer in the device structure of the present invention is not particularly limited. Generally, it is desirable that this layer is used in the composition and pH range with physiologically less stimulation to the attached site on the skin or mucous membrane etc. Further, it is more desirable that the electrolyte composition and pH are used in consideration of the isoelectric points of the substances to be detected, that is, marker proteins, peptides, etc. in order to move substances to be detected to the device for diagnosis by the iontophoresis. Further, as a holding method of the conductive layer used in the present invention, a matrix type or reservoir type or the like represented as gel type or solution type is not particularly limited. Conductive gel of such a matrix type, for example, includes: agar, gelatin, xanthan gum, locust bean gum, carrageenan, gellan gum, tamarind gum, curdlan, pectin, farceleran, guar gum, alginic acid, sodium alginate, tara gum, karaya gum, cellulose, and their derivatives; synthetic polymers and copolymers such as polyacrylic acid, polyvinyl pyrrolidone, polyvinyl alcohol, and polyisobutylene to be used each alone, or in combination of two or more of them. As a holding means of a reservoir type electrolyte represented as the solution type, a member material having various porous or capillary structures (hereinafter, sometimes simply referred to as porous substance) is used. As the porous substances, organic porous substances (e.g. natural fibers such as cellulose; semisynthetic fibers such as cellulose acetate; fiber aggregates formed with synthetic fibers such as polyethylene, polypropylene, nylon and polyester; sheets such as paper; fabrics such as woven fabric and nonwoven fabric; porous synthetic resins such as porous polypropylene, porous polystyrene, porous poly(methyl methacrylate), porous nylon, porous polysulfone and porous fluoride resin; water-absorbing resins such as sponge, and the like); inorganic porous substances (e.g. porous ceramic form; ceramics having porous substances or capillary structures, and the like) and the like may be used. To a holding means of them, if necessary, an electrolyte, pH regulator, stabilizer, thickner, wetting agent, surfactant, solubilizer, or the like may be added or impregnated.

The member for detection of the device structure according to the present invention can preferably adsorb and fix living tissues, blood or cells, or a substance secreted from those, and more preferably can retain the adsorbability when charging an electric field. In addition, the member for detection desirably is hydrophilic, has high adsorbability to a protein, a peptide and the like, and is stable to water, alcohol, formaldehyde etc. used for detecting the adsorbed and fixed substances so that they are not detached from the member for detection and not dissolved in those solvents. Although such a member for detection is not particularly limited, various hydrophilic porous substances having porous or capillary structures highly adsorbable to a protein and a peptide, or hydrophilically treated hydrophobic porous substances, may be used.

The preferred member for detection can include organic porous substances (e.g. natural fibers such as cellulose; semisynthetic fibers such as nitrocellulose and cellulose acetate; fiber aggregates formed with synthetic fibers such as polyethylene, polypropylene, nylon and polyester; sheets such as paper; fabrics such as woven fabric and nonwoven fabric; porous synthetic resins such as porous polypropylene, porous polystyrene, porous poly (methyl methacrylate), porous nylon, porous polysulfone, porous fluoride resin, and porous polyvinylidene difluoride).

The member for detection used in the present invention has a characteristic of showing high absorbability to proteins, peptides and the like, and the large adsorbing amount. It is desirable that the adsorbing amount of proteins to the member for detection is 20µg per cm² or more, preferably 40µg per cm² or more, more preferably 50µg per cm² or more. Herein, the adsorbing amount of proteins can be calculated by a simply measurable conventional method using typical proteins in which e.g. after immersing the member for detection of 1.3 cm² under room temperature for 90 min in 400µl of a solution (phosphate buffer solution of 1 mg/ml as protein concentration) containing a tracer amount of bovine serum albumin or human insulin labeled with ¹²⁵I, it is washed with the phosphate buffer solution and the residual radioactivity is measured. Further, the adsorbing amount may be calculated using the desired substances to be detected.

These members for detection have porous structures and the pore size can be selected from the range acceptable to the living body if detection performance is not damaged in the measurement of holding amount of the substance to be detected and of the desired substance. The average pore size is, for example, 0.001-20µm, preferably about 0.01-10µm, more preferably about 0.01-1µm.

Thickness of the member for detection can be selected from the range acceptable to the living body if detection performance is not damaged in the measurement of holding amount of the substance to be detected and of the desired substance, and is desirably about 0.1-500µm, preferably about 1-300µm, more preferably about 5-200µm.

Then, the detecting method according to the present invention will be described. According to the device structure of the present invention, living tissues, blood or cells or secreted substance from them adsorbed and fixed onto the member for detection are tumor-related antigens, tumor markers or other tumor-related substances. Staining or measuring of such member for detection by the immunological or chemical method enables diagnoses of tumor and the like. Detectable tumors by the device structure and the detecting method of the present invention can include skin cancer, oral cancer, breast cancer, rectal cancer and the like. Skin cancers can include malignant melanoma, basal cell cancer, epidermoid cancer and the like, and oral cancers can include tongue, cancer, buccal mucosa cancer, laryngeal cancer and the like.

In addition, the above described tumor-related antigens, tumor markers or other tumor-related substances can be selected from melanoma cells, melanoma marker (NKI/C3), melanoma marker (PAL/M1), melanoma markers (S-100 α and β), carcinoembryonic antigen (CEA), neuroblastomas (CE7), neuroblastomas (AD2), malignin, α-fetoproteln (AFP), pepsinogen, basic fetoprotein (BFP), pancreas carcinoembryonic antigen (POA), embryonic prealbumin (EPA), carbohydrate antigen (CA19-9), pancreatic cancer-related antigen (CA50), cancer antigen (CSLEX-1), pancreatic cancer-related antigen (sialylSSEA-1), pancreatic cancer-related antigen (Dupan-2), cancer antigen (NCC-ST-439), carbohydrate antigen (sialylTn), cancer antigen (CA72-4), cancer antigen (KMO-1), pancreatic cancer-related antigen (SPan-1), carbohydrate antigen (CA125), cancer antigen (CA15-3), planocellular carcinoma (SCC), seminoprotein (γ-Sm), prostatic specific antigen (PA), ferritin, tissue polypeptide antigen (TPA), tumor-related antigen (CYFRA-21-1), acidic immunoprotein (IAP), immunosuppressive acidic protein, prostatic acidic protein (PAP), neuron specific enolase (NSE), chorionic gonadotropin (hCG), enzyme, amino acid (specifically Ala, Glu), mucosa containing mucopolysaccharide, dopa, dopamine and hormones.

Considering specificity and sensitivity in detection of the melanoma markers, a variety of melanoma antibodies can be used. Any melanoma antibodies may be used if they have high specificity to malignant melanoma. In the device structure for diagnosis of the present invention which detach and fix or adsorb and fix tissues, blood or cells, etc. by being attached onto the lesion site, it can be detected whether or not the tumor markers exist in a cell staining mode in the tissues, blood or cells, using the EIA method with the above described antibodies, immunoassay with a variety of labeled particles (latex, gold colloid, etc.) and fluorescence immunoassay. Also in the case of 5-S-cystenyldopa, the detection can be performed using a fluorescence microscope after treating the member for detection of the device structure for diagnosis of the present invention with formaldehyde gas. In such a case, the judgement can be qualitatively performed by depth of the color of the stained cells and their number. Using any of the known detecting methods other than the above described methods, the tissues, cells, proteins, peptides, etc. which are adsorbed and fixed by the device structure of the present invention can be also measured.

Although Examples and Comparative Examples will be described more in detail based on the following Experimental Examples, the present invention is not limited to these Examples. In Experimental Examples 1-3 adsorbing characteristics and diffusibility to proteins and holding performance in the electric field of the member of detection used in the device for diagnosis are examined. In Experimental Example 4, after selecting the member for detection of the device for diagnosis in Experimental Examples 1-3 evaluated was in influence on staining performance by the immunological staining method to a human carcinoembryonic antigen (human CEA). Further, in Experimental Example 5, detection from the skin of human CEA which is a breast tumor marker was examined using the iontophoresis device for diagnosis of the present invention. In addition, bovine serum albumin (BSA, manufactured by Sigma Co., Ltd.), human insulin (manufactured by Sigma Co., Ltd.) and human CEA (manufactured by Biochemical Industry Co., Ltd.) were adopted as a typical protein, peptide and marker examples for diagnosis.

### (Experimental Example 1)

To evaluate characteristics of the member for detection in the device for diagnosis, adsorption characteristics to proteins were examined after selecting porous membranes composed of different materials. Herein, the adsorbing amount of proteins was calculated by a simply measurable conventional method using typical proteins in which after immersing the member for detection of 1.3 cm² under room temperature for 90 min in 400µl of a solution (phosphate buffer solution of 1 mg/ml as protein concentration) containing a tracer amount of bovine serum albumin (¹²⁵I-BSA, 0.827 mCi/mg, ICN Pharmaceutical Inc.) or human insulin (0.912 µCi/µg, ICN Pharmaceutical Inc.) labeled with ¹²⁵I, it was washed with the phosphate buffer solution and the residual radioactivity was measured using a γ-counter. The materials of the member for detection used in each Example and Comparative Example are shown as follows.
Example 1: Nitrocellulose membrane (Nitrocellulose Membrane manufactured by Biolad)
Example 2: Nylon membrane (Biodyne A, manufactured by Pall Ltd.)
Example 3: Hydrophobic polyvinylidene difluoride (PVDF) membrane (PVDF Membrane, manufactured by Biolad)
Comparative Example 1: Hydrophilic polyvinylidene difluoride (PVDF) membrane (Hydrophilic Durapore, manufactured by Millipore Inc.)

Herein, the hydrophobic PVDF membrane, it was immersed in 100% methanol for several sec. as a hydrophilic treatment prior to the test followed by immersing it in a phosphate buffer solution.

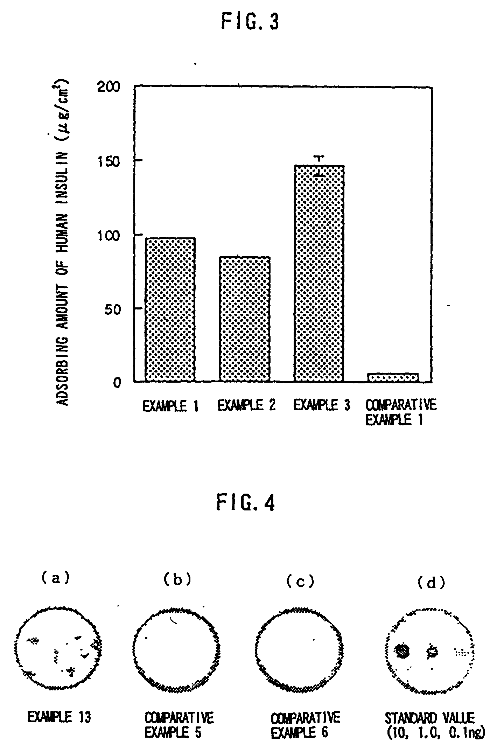

FIG. 2 is a graph showing absorbability of bovine serum albumin (BSA) to the member for detection used in the device for diagnosis in Examples 1-3 and Comparative Example 1(mean ± standard deviation, n = 3). FIG. 3 is a graph showing absorbability of human insulin to the member for detection used in the device for diagnosis similarly in Examples 1-3 and Comparative Example 1 (mean ± standard deviation, n = 3). The adsorbing amount of BSA, as shown in FIG. 2, was about 80µg/cm² for Example 1, about 50µg/cm² for Example 2, and about 170µg/cm² for Example 3. For Comparative Example 1 using as a member of lower absorbability to proteins, it was not more than 5µg/cm². On the other hand, the adsorbing amount of human insulin, as shown in FIG. 3, was about 100µg/cm² for Example 1, about 80µg/cm² for Example 2, and about 150µg/cm² for Example 3. For Comparative Example 1, it was 5µg/cm² or less. Nitrocellulose, nylon and hydrophobic PVDF showed high absorbability to BSA and human insulin which are a typical protein and peptide.

### (Experimental Example 2)

Then, diffusibility of each protein on different members for detection was examined. Using the same method as that of Experimental Example 1: after immersing the member for detection of 1.3 cm² under room temperature for 90 min in 400µl of a solution (phosphate buffer solution of 1 mg/ml as protein concentration) containing a tracer amount of bovine serum albumin or human insulin labeled with ¹²⁵I, it was washed with the phosphate buffer solution and the residual radioactivity was measured using a γ-counter. Thereafter, it was further immersed in 1 ml of the phosphate buffer for 24 hr. and similarly the residual radioactivity was measured using a γ-counter. The materials of the member for detection used in each Example and Comparative Example are shown as follows.
Example 4: Nitrocellulose membrane (Nitrocellulose Membrane, manufactured by Biolad)
Example 5: Nylon membrane (Biodyne A, manufactured by Pall Ltd.)
Example 6: Hydrophobic PVDF membrane (PVDF Membrane, manufactured by Biolad)
Comparative Example 2: Hydrophilic PVDF membrane (Hydrophilic Durapore, manufactured by Millipore Inc.)

Herein, the hydrophobic PVDF membarne was hydrophilically treated prior to the test similarly to the above described Experimental Example 1.

The results obtained from Examples 4-6 and Comparative Example 2 are shown in Table 1.

**Table 1**

| Experimental Example 2 | Initial adsorbing Amount (µg/cm²) | | Residual Adsorbing Amount (µg/cm²) after 24 Hr | |
|---|---|---|---|---|
| | Mean | Standard Deviation | Mean | Standard Deviation |
| Example 4 | 83.97 | 1.45 | 80.71 | 1.47 |
| Example 5 | 55.16 | 1.51 | 50.99 | 1.77 |
| Example 6 | 138.56 | 7.39 | 137.20 | 7.33 |
| Comparative Example 2 | 1.22 | 0.11 | 0.60 | 0.14 |

Table 1 shows diffusibility of bovine serum albumin to the member for detection used in the device for diagnosis (mean ± standard deviation, n=3). As shown in Table 1, reduction of the adsorbing amount after 24 hr in Examples 4, 5 and 6 each was scarcely observed, and it was confirmed that the adsorbed protein was not almost diffused in the solvent. In Comparative Example 2, the initial adsorption was scarcely observed, and the adsorbing amount after 24 hr was further reduced.

### (Experimental Example 3)

The adsorbing performance of the member for detection in the device for diagnosis, particularly the characteristics in the electric field was examined. Using BSA and human insulin as typical proteins and further human CEA as one of markers for diagnosis, the adsorbing performance in the electric fields of the different members for detection was examined. Electrophoresis of sodium lauryl sulfate (SDS)-polyacrylamide gel in 100-150 V was conducted (10-25% polyacrylamide gradient gel was used) for BSA, human insulin and human CEA. Herein, the applied amount for electrophoresis of each protein was set as 2.5µg. Two sheets of transfer membranes shown in the Examples and Comparative Examples were overlapped on the gel after electrophoresis, further the third sheet of nitrocellulose membrane was placed onto them as a support membrane and transcription was performed at 150 mA for 3 hr. The transferred protein was stained with amide black staining solution. The materials of the member for detection used in each Example and Comparative Example are shown as follows.
Example 7: Nitrocellulose membrane (Nitrocellulose Membrane, manufactured by Biolad)
Example 8: Nylon membrane (Biodyne A, manufactured by Pall Ltd.)
Example 9: Hydrophobic PVDF membrane (PVDF Membrane, manufactured by Biolad)
Comparative Example 3: Hydrophilic PVDF membrane (Hydrophilic Durapore manufactured by Millipore Inc.)

Herein, the hydrophobic PVDF membarne was hydrophilically treated prior to the test similarly to the above described Experimental Examples 1 and 2.

The results obtained from Examples 7-9 and Comparative Example 3 are shown in Table 2.

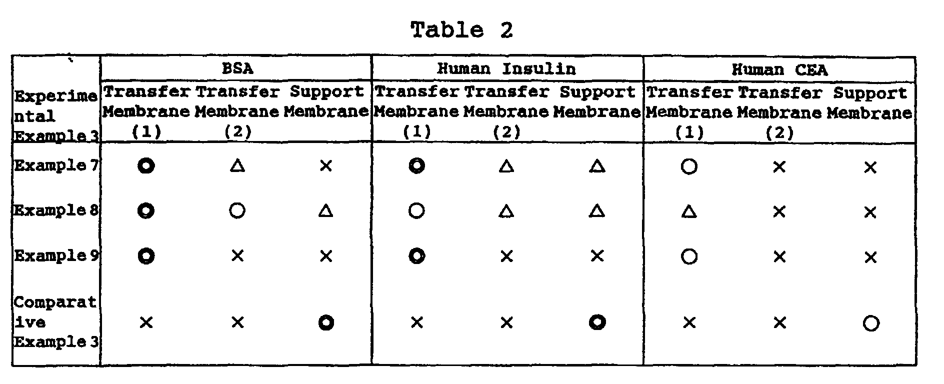

Table 2 shows the holding ability of bovine serum albumin, human insulin and human carcinoembryonic antigen in the electric fields to the member for detection used in the device for diagnosis. As shown in Table 2, the BSA (molecular weight: about 60,000) is held mainly on the transfer membrane (1) in Examples 7-9, while it was slightly detected on transfer membrane (2). Similarly to BSA the human insulin (molecular weight: about 6,000) was likely to be held mainly on the transfer membrane (1), while it was slightly detected on the transfer membrane (2). Further, for human CEA (molecular weight: about 180,000), it was held on the transfer membarane (1) and not detected on the transfer membrane (2). On the other hand, BSA, human insulin and human CEA in Comparative Example 3 were neither adsorbed nor held at all since they passed on the transfer membranes by charging the electric field. Consequently, nitrocellulose, nylon and hydrophobic PVDF showed high adsobability and low diffusibility to any of proteins and peptides, and strong holding ability In the electric field.

### (Experimental Example 4)

Next, influence of different members of detection on immunological staining of the human CEA was examined. After the human CEA of 0.1, 1.0 and 10 ng each was dropped onto a variety of members for detection and dried to fix, the following immunological staining was performed. After blocking operation of a variety of members of detection with the human CEA fixed, they were immersed in the primary antibody solution (mouse antihuman CEA monoclonal antibody, clone EB-016, manufactured by Japan Biotest Laboratory). After washing with borate buffer solution, they were immersed in the biotin labeled secondary antibody solution (biotin labeled antimouse IgF(ab')², manufactured by DAKO JAPAN Co., Ltd.). After washing, they were immersed in peroxidase labeled streptoavidin solution (manufactured by Biochemical Industry Co.. Ltd.), washed with borate buffer solution, and colored using DAB (diaminobenzidinetetrahydrochloride) as a substrate. The materials of the member for detection used in each Example and Comparative Example are shown as follows.
Example 10: Nitrocellulose membrane (Nitrocellulose Membrane, manufactured by Biolad)
Example 11: Nylon membrane (Biodyne A, manufactured by Pall Ltd.,)
Example 12: Hydrophobic PVDF membrane (PVDF Membrane, manufactured by Biolad)
Comparative Example 4: Hydrophilic PVDF membrane (Hydrophilic Durapore, manufactured by Millipore Inc.)

Herein, the hydrophobic PVDF membarne was hydrophilically treated prior to the test similarly to the above described Experimental Examples 1 and 2.

The results obtained from Examples 10-12 and Comparative Example 4 are shown in Table 3.

**Table 3**

| Experimental Example 4 | Stainability of Human CEA | | |
|---|---|---|---|
| | 0.1 ng | 1 ng | 10 ng |
| Example 10 | △ | ○ | ⓞ |
| Example 11 | △ | ○ | ⓞ |
| Example 12 | X | △ | ○ |
| Comparative Example 4 | X | X | X |

Table 3 shows influence of each member for detection on staining performance by the immunological staining method to the human carcinoembryonic antigen (human CEA). As shown in Table 3 in Examples 10 and 11, the human CEA of 1 ng or more was detectable. In Example 12, the human CEA of 10 ng or more was detectable, while that of 1 ng or less was not detected. In Comparative Example 4, it was not stained at all since it presumably flowed out in an operation process of the immunological staining.

### (Experimental Example 5)

Detection of human CEA from the skin was examined in vitro using the device according to the present invention. Herein, epidermal sheets of Yucatan micropigs were used as a skin model. A device for diagnosis (anode: silver electrode) was applied onto the horny layer side, and a nonwoven fabric layer added with the human CEA (10µg/ml, phosphate buffer solution (pH = 7.4)) was arranged onto the epidermis side, and further a reference electrode (cathode: silver chloride electrode) was correspondingly connected. The nylon membrane (Biodyne A, manufactured by Pall Ltd.) showing high adsorbability, low diffusibility, high holding ability and high stainability was used as the member for detection of the device for diagnosis. Energizing with current was conducted at 50 kHz of frequency and at 0.5 mA/cm² in a pulse direct voltage of a 50% on/off ratio for 1 hr. Immunological detection was performed by the staining method similar to that of the above described Experimental Example 4. In addition, the human CEA of 0.1, 1.0, 10 ng each was dropped onto the member for detection to define the standard values.
Example 13: In the case where the human CEA was added and the iontophoresis was conducted.
Comparative Example 5: In the case where the human CEA was added and the iontophoresis was not conducted.
Comparative Example 6: In the case where the iontophoresis was conducted using only the phosphate buffer solution.
The results obtained in Example 13 and Comparative Examples 5 and 6 are shown in Table4.

**Table 4**

| Experimental Example 5 | Stainability | Concentration of Detected Human CEA |
|---|---|---|
| Example 13 | ○ | 10 ng or more |
| Comparative Example 5 | X | 0.1 ng or less |
| Comparative Example 6 | X | 0.1 ng or less |

Table 4 shows concentration of the human CEA and stainability detected from the skin of human carcinoembryonic antigen (human CEA) using the iontophoresis device for diagnosis of the present invention. Further, FIGS. 4 (a)-(d) are a graph showing the results of the immunological staining of carcinoembryonic antigen (human CEA) on the member for detection. As shown in Table 4 and FIGS. 4 (a)-(d), the human CEA was detected on several positions of the member for detection in Example 13 and 10 ng or more of the human CEA was detected judging the detected amount compared with the standard values. The staining results suggested that the human CEA was induced on the member for detection through intraspidermal pores and sweat glands in Yucatan micropigs, while in Comparative Examples 5 and 6, no human CEA was detected (0.1 ng or less of the standard value). Thus, the tumor markers were detected even through the skin, and usefulness of the present device structure for diagnosis and the staining method were shown.

Thus, the device structure using the iontophoresis provided by the present invention is useful for diagnoses of tumors and the like. If the device structure of the present invention is used, the samples can be collected extremely readily in short time without requiring surgical excision or paracentesis suction of the surface of the skin or mucous membrane and without giving patients pain. Therefore, it enables the mass health examination for skin cancer, oral cancer, breast cancer, rectal cancer and the like. Further, it can reduce the recurrence cases caused presumably by surgical measures and the like. The early diagnosis of tumors can be also performed even for tumors in extremely early stages which are difficult to be judged, since it can be confirmed whether tumor-related antigens, tumor-related markers or other tumor-related substances exist or not by use of the device structure of the present invention. Furthermore, according to the device structure of the present invention, the diagnoses can be performed rapidly and readily compared with the conventional arts and therefore are economically useful because the tissues and cells of the surface of the skin or mucous membrane, or a substance secreted from them or other substances are adsorbed with fixation on the member for detection and these can be directly used as samples,

### INDUSTRIAL APPLICABILITY

The iontophoresis device structure and the detecting method according to the present invention are useful for detecting a physiological substance rapidly and in high sensitivity, being suitable for application to the iontophoresis in the medical field.

## Claims

1. An iontophoresis device structure comprising an electrode for an iontophoresis, a member for detection constituted so as to adsorb a physiological substance by the iontophoresis, and a conductive layer arranged between said electrode and said member for detection.

2. The iontophoresis device structure according to claim 1, said member for detection is composed of a porous membrane.

3. The iontophoxesis device structure according to claim 1 or 2, adsorbability of protein to said member for detection is 20µg per cm² or more.

4. The iontophoresis device structure according to any of claims 1-3, thickness of said member for detection is 5-200µm.

5. The iontophoresis device structure according to any of claims 1-4, said member for detection is constituted so as to adsorb at least one of living tissues, blood and cells.

6. The iontophoresis device structure according to any of claims 1-4, said member for detection is constituted so as to adsorb a substance secreted from at least one of living tissues, blood and cells.

7. The iontophoresis device structure according to claim 6, said secreted substance is a peptide or a protein.

8. The iontophoresis device structure according to any of claims 1-4, said member for detection is constituted so as to adsorb a tumor-related antigen, a tumor marker or other tumor-related substance.

9. The iontophoresis device structure according to claim 8, said tumor-related antigen, tumor marker or other tumor-related substance is selected from groups composed of melanoma cells, melanoma marker (NKI/C3), melanoma marker (PAL/M1), melanoma markers (S-100 α and β), carcinoembryonic antigen (CEA), neuroblastoma (CE 7), neuroblastoma (AD2), malignin, α-fetoprotein (AFP), pepsinogen, basic fetoprotein (BFP), pancreas carcinoembryonic antigen (POA), embryonic prealbumin (EPA), carbohydrate antigen (CA19-9), pancreatic cancer-related antigen (CA50), cancer antigen (CSLEX-1), pancreatic cancer-related antigen (sialylSSEA-1), pancreatic cancer-related antigen (Dupan-2), cancer antigen (NCC-ST-439), carbohydrate antigen (sialylTn), cancer antigen (CA72-4), cancer antigen (KMO-1), pancreatic cancer-related antigen (SPan-1), carbohydrate antigen (CA125), cancer antigen (CA15-3), planocellular carcinoma (SCC), seminoprotein (γ-Sm), prostatic specific antigen (PA), ferritin, tissue polypeptide antigen (TPA), tumor-related antigen (CYFRA-21-1), acidic immunoprotein (IAP), immunosuppressive acidic protein, prostatic acidic protein (PAP), neuron specific enolase (NSE), chorionic gonadotropin (hCG), enzyme, amino acid, mucosa containing mucopolysaccharide, dopa, dopamine and hormones.

10. An applicator for an iontophoresis comprising a backing having a concave, an electrode arranged in the bottom of said backing concave, a member for detection of a physiological substance arranged in the top of said backing concave, and a conductive layer arranged between said electrode and said member for detection of a physiological substance.

11. The applicator for the iontophoresis according to claim 11, further comprising an adhesive sheet having a opening in a part corresponding to said member for detection of a physiological substance.

12. A method for detecting physiological substances comprising the steps of: adsorbing a physiological substance onto a member for detection using an iontophoresis; and detecting a physiological substance adsorbed onto said member for detection by an immunological or chemical method.

13. The method for detecting physiological substances according to claim 12, said detection is performed by staining or measuring of said physiological substance.

14. An iontophoresis system comprising: an iontophoresis device including a member for detection which is constituted so as to adsorb a physiological substance by an iontophoresis, an electrode for a device, and a conductive layer arranged between the electrode for the device and the member for detection; a reference device including a reference electrode corresponding to the electrode for the device; and a power source connecting electrically between the electrode for the device and the reference electrode.

15. The iontophoresis system according to claim 14, said member for detection has a porous structure having an average pore size of 0.001-20µm.
